# EUROPEAN PATENT SPECIFICATION

(11) **EP 3 731 886 B1**
(45) Date of publication and mention of the grant of the patent: **05.07.2023**
(21) Application number: 18877314.7
(22) Date of filing: 26.12.2018
(51) Int. Cl.: A61L 27/12, A61K 33/42, C01B 25/32, C04B 35/447, A61L 27/58, C04B 35/64

(54) **PRODUCTION OF BETA-TRI-CALCIUM PHOSPHATE (BETA-TCP) WITH HIGH PURITY**
HERSTELLUNG VON BETA-TRI-CALCIUM-PHOSPHAT (BETA-TCP) MIT HOHER REINHEIT
PRODUCTION DE BÊTA-TRI-CALCIUM PHOSPHATE (BÊTA-TCP) DE GRANDE PURETÉ

(30) Priority: 27.12.2017 TR 201722287; 03.12.2018 TR 201818446
(43) Date of publication of application: 04.11.2020
(73) Proprietor: Betatech Medikal Cihazlar Sanayi Mümessillik iç ve Dis Ticaret Ltd. Sti., Istanbul (TR)
(72) Inventor: HAZARYORUC, Afife Binnaz, Istanbul (TR); TORAMAN, Abdullah, Besiktas/Istanbul (TR)
(74) Representative: Kaya, Erdem
(86) International application number: PCT/TR2018/050908
(87) International publication number: WO 2019/132843

(56) References cited:
- EP-A2- 1 394 132
- US-A1- 2009 191 111
- US-B2- 7 901 650

## Description

### TECHNICAL FIELD

The invention is a method for the production of beta-tricalcium phosphate.

### KNOWN STATE OF THE ART

Bone and tooth tissues in human body can be damaged due to accident, disease and aging. Therefore, the damaged bones may have to be renewed or changed. Inorganic structure of bone is hydroxyapatite and its basic elements are calcium and phosphate. Hydroxyapatite is joined with collagen and a very strenght and flexible structural material is obtained and forms an intravenous dense, metabolically active and viable bone tissue.

Today, hydroxyapatite is used in a stand-alone manner or is used as bone filling substance in regions which do not bear load together with auto-graft. However, in orthopedics and traumatology, in cases where artificial bone tissue is desired to be rapidly dissolved, bio-ceramic materials, which are similar to the composition and structure of the bone tissue and known as β-TCP (beta-tri-calcium phosphate), are used in a stand-alone manner, and bio-ceramic powder, produced in a similar characteristic to the composition of the bone tissue, is the suitable solution for the repair of the defects which occur in the bone tissue.

The minimum criterion in preventing the complications which occur after surgical process and in forming healthy bone tissue is that the purity of β-TCP (Beta-tri-calcium phosphate) powder is greater than 95% and the heavy metal proportions are such that As: ≤0.5 mg/kg, Hg: ≤0.5 mg/kg, Pb: ≤5 mg/kg. In the present art, there are products which fulfill the defined criteria; however, even these products may lead to important complications after the surgical process. For increasing healthy formation proportion of the bone tissue, purity of β-TCP is increased and the heavy metal proportion is reduced to levels which are lower than the abovementioned values. Since the products with these characteristics do not exist in the medical market, alternative surgical applications cannot be realized.

Another usage area of tri-calcium phosphate is the food sector. Tri-calcium phosphate, which can be used for enriching food products with low cost, also functions as absorbent and as agent which prevents agglomeration and as buffering agent. Tri-calcium phosphate is moreover used for increasing fluidity in foods and as baking agent.

In the known state of the art, there are applications related to synthesizing of the β-TCP (beta-tri-calcium phosphate) powder. One of these is the US patent with number US20030235622A1. In the application, synthesizing of alpha- and beta-tri-calcium phosphate (TCP) powders by using wet chemical method in order to be used as raw substance in artificial bones, artificial joints, and artificial tooth roots and calcium phosphate-based bioceramics is disclosed. In said invention, calcium nitrate tetra-hydrate and di-ammonium hydrogen phosphate are used. However, said application relates to a method which is suitable for use in tri-calcium phosphate production having only sub-micron particle size and however, there is no disclosure related to that the tri-calcium phosphate obtained by means of the method disclosed in this application has higher purity. Thus, in the known state of the art, there is no description which will guide the skilled person to obtain high purity tri-calcium phosphate.

EP1394132 A2 patent number of invention, describes a new calcium phosphate cement powder, whose composition can best be described over the Ca/P molar ratio range of 1.35 to 1.40, most preferably 1.39, and whose two components were prepared by wet chemical synthesis procedures. One component is chemically-synthesized, bi-phasic alpha-TCP (Ca₃(PO₄)₂, 95 wt%) + HA (Ca₁₀(PO₄)6(OH)₂, 5wt%) powder, while the second component is again a chemically-synthesized, single-phase DCPD (CaHPO₄·2H₂O) powder. A setting solution (3 wt% Na₂HPO₄·2H₂O dissolved in distilled water) is used to form a self-setting calcium phosphate cement from the powder mixture. This cement can be used as bone filler or bone substitute in applications, which require higher rates of resorption.

US7901650 B2 patent number of invention, a method of making a porous tricalcium phosphate, said method comprising: combining a tricalcium phosphate with graphite to produce a graphite containing tricalcium phosphate composition; and sintering said graphite containing tricalcium phosphate composition to produce said porous tricalcium phosphate.

US20091191111 A1 patent number of invention, relates to a method of preparing highly sinterable calcium phosphate-based ceramic powder and a sintered compact thereof and, more particularly, to a method of preparing highly sinterable and nano-sized hydroxyapatite (HAp) powder or β-tricalcium phosphate (β-TCP) powder, which can be usefully used as a bone substitute material because it is highly densified, and a sintered compact thereof.

As a result, because of all of the abovementioned problems, an improvement is required in the related technical field.

### OBJECT OF THE INVENTION

The present invention relates to high purity beta-tri-calcium phosphate (Beta-TCP) production, for eliminating the above mentioned disadvantages and for bringing new advantages to the related technical field.

The object of the present invention is to realize β-TCP production by using calcium deficient hydroxyapatite (CDHA) synthesized by means of the chemical precipitation method.

The object of the present invention is to realize production of β-TCP powder with high purity (over 99%) from calcium deficient hydroxyapatite (CDHA) by using optimized reaction steps and sintering method.

The object of the present invention is to increase β-TCP (beta-tri-calcium phosphate) powder purity over 95% which is the criterion value determined in formation of healthy bone tissue and in prevention of the complications which will occur after surgical process.

The subject of the invention is a method for the production of beta-tricalcium phosphate. The production method subject to the invention includes the following process steps;
a. Preparing Ca(OH)₂ solution by adding distilled water onto Ca(OH)₂,
b. Bringing reaction temperature to 25-35°C,
c. Adjusting reaction pH to the range between 7-9 by using HCl acid,
d. Adding distilled water onto [(NH₄)₂HPO₄] and providing completely dissolving and preparing [(NH₄)₂HPO₄] solution,
e. Adding [(NH₄)₂HPO₄] solution to Ca(OH)₂ solution,
f. Bringing reaction pH to between 7-9 by means of NH₄OH solution and mixing and ending the reaction afterwards,
g. Leaving the obtained solution to aging for between 8 and 30 hours,
h. Adding distilled water onto [(NH₄)₂SO₄] and preparing 3% [(NH₄)₂SO₄] solution,
i. Decanting the aged solution and adding [(NH₄)₂SO₄] solution and leaving for aging for 8-30 hours without mixing,
j. Filtering the obtained solution and washing with plenty of water such that the pH of the solution becomes between approximately 6 and 8,
k. Drying the obtained humid powder in the stove,
l. Sintering the dried powder for 1 hour at a temperature between 900 and 1500°C,
m. Obtaining the final product by instantaneous cooling of the product at the end of the sintering process.

In an application of the present invention, in step e), [(NH₄)₂HPO₄] solution is added to Ca(OH)₂ solution with flow rate of 3-5 ml/min, for instance, 3 ml/min or 4 ml/min or 5 ml/min.

In an application of the present invention, in step f), the solution whose pH value is adjusted with NH₄OH is mixed 15-30 minutes.

In an application of the present invention, in steps g) and i), the solution is left for resting for duration of 12-28 hours.

In an application of the present invention, in step i), 2-4% [(NH₄)₂SO₄] solution is used.

In an application of the present invention, the powder obtained in step k) is dried in stove at a temperature between 70 and 100°C.

The beta-tri-calcium phosphate (β-TCP) obtained by means of said method has at least 99% purity in a different manner from the beta-calcium tri-phosphates (β-TCP) obtained by means of the methods already present in the related art. Moreover, the impurities existing in said beta-calcium tri-phosphate differentiate from the known art by having impurity (Pb ≤0.077, As ≤0.016, Hg ≤0.015) which is substantially lower than the impurity criterion (As: ≤0.5 mg/kg, Hg: ≤0.5 mg/kg, Pb: ≤5 mg/kg) which is already present in the related art.

The structural and characteristic properties and all advantages of the present invention will be understood in a clearer manner thanks to the detailed description given below and therefore, evaluation shall be made by taking into consideration this detailed description.

### DETAILED DESCRIPTION OF THE INVENTION

In this detailed description, the method of production of beta-tri-calcium phosphate (β-TCP) with high purity, which has an osteoconductive support matrix which can be resorbed and which is bio-compliant when implanted to the defect area and which provides new bone formation in the defect area and which is resorbed while displacing with the newly formed bone in time, is explained with references to examples without forming any restrictive effect only in order to make the subject more understandable.

In the production of the subject matter high purity beta-tri-calcium phosphate (β-TCP), calcium hydroxide (calcium source) and di-ammonium hydrogen phosphate (phosphate source) are used as the starting substance, and the usage amounts of the starting substances are determined in a stoichiometric manner such that Ca/P proportion is 1.5. The reaction pH is fixed to 7-9 by means of the ammoniac solution and hydro-chloric acid.

Method of production of beta-tri-calcium phosphate (β-TCP) with high purity:
- 100 ml distilled water is added onto 0.08 mol Ca(OH)₂ and the Ca(OH)₂ solution is prepared,
- Reaction temperature is brought to 30°C and mixing is continued,
- Reaction pH is adjusted to 7-9 by using HCl acid,
- 100 ml distilled water is added onto 0.053 mole [(NH₄)₂HPO₄] and by providing complete dissolving, [(NH₄)₂HPO₄] solution is prepared,
- [(NH₄)₂HPO₄] solution is added to the Ca(OH)₂ solution such that 3-5 ml is added in a minute (3-5 ml/minute),
- The reaction pH is again brought to between 7-9 by means of NH₄OH solution and is mixed for 20 minutes and the reaction is ended at the end of the duration,
- The obtained solution is left for aging for 16 hours,
- 200 ml distilled water is added onto 6 grams of [(NH₄)₂SO₄] and 3% [(NH₄)₂SO₄] solution is prepared,
- The aged solution is decanted and 3% [(NH₄)₂SO₄] solution is added thereon and is again left for aging for 24 hours without mixing,
- The obtained solution is filtered by using gooch crucible por:4 and is washed with plenty of water such that pH is approximately 7,
- The obtained humid powder is dried in stove for 24 hours at 90°C,
- The dried powder is sintered for 1 hour at 1100°C,
- The product is instantaneously cooled at the end of the sintering process.

The beta-tri-calcium phosphate obtained by means of the subject matter method has white powder form and its purity is ≥99, crystal particle size is 73.83 nanometers, size distribution is 1-1000 micrometers, density is 2.973 gr/cm3.

The impurity proportion of beta-tri-calcium phosphate (heavy metals) is such that Pb ≤0.077, As ≤0.016, Zn≤ 0.076, Hg ≤0.015, Cr ≤0.085, Co≤ 0.07, Ni ≤0.02, Cd ≤0.004, Cu≤ 0.13.

The most important point in tissue engineering is to form response for healing of the bone in the defect region. Another important point is that the bone is structurally integrated with the peripheral bone tissue as a result of re-shaping of the bone. Within this scope, the beta-tri-calcium phosphate powder, synthesized by means of the subject matter method, is chemically held onto the surface of the bone tissue (osteointegration) such that no fibrosis tissue is formed in between, supports new bone formation (osteoconduction), stimulates transformation to osteoblastic phenotype in the peripheral tissue (osteoinduction) and forms the new bone cells in the transferred area of the powder (osteogenesis). Moreover, in order for the beta-tri-calcium phosphate powder synthesized by means of the subject matter method to stimulate the new bone cell, the impurity proportion and the chemical composition are substantially important.

Beta-tri-calcium phosphate, synthesized by means of the subject matter method, can also be used as absorbent in the food sector, as baking agent, as agent increasing fluidity and as agent preventing agglomeration in foods.

## Claims

1. A method to be used in beta-tri-calcium phosphate production, said method is **characterized by** comprising the steps of:
a. Preparing Ca(OH)₂ solution by adding distilled water onto Ca(OH)₂,
b. Bringing reaction temperature to 25-35°C and preferably, to 30°C and continuing mixing,
c. Adjusting reaction pH to the range between 7-9 by using HCl acid,
d. Adding distilled water onto [(NH₄)₂HPO₄] and providing completely dissolving and preparing [(NH₄)₂HPO₄] solution,
e. Adding [(NH₄)₂HPO₄] solution to Ca(OH)₂ solution,
f. Bringing reaction pH to between 7-9 by means of NH₄OH solution and mixing and ending the reaction afterwards,
g. Leaving the obtained solution to aging for between 8 and 30 hours,
h. Adding distilled water onto [(NH₄)₂SO₄] and preparing 3% [(NH₄)₂SO₄] solution,
i. Decanting the aged solution and adding [(NH₄)₂SO₄] solution and leaving for aging for 8-30 hours without mixing,
j. Filtering the obtained solution and washing with plenty of water such that the pH of the solution becomes between approximately 6 and 8, preferably 7,
k. Drying the obtained humid powder in the stove,
. Sintering the dried powder for 1 hour at a temperature between 900 and 1500°C,
m. Obtaining the final product by instantaneous cooling of the product at the end of the sintering process.

2. The method according to claim 1, **wherein** in step e), [(NH₄)₂HPO₄] solution is added to Ca(OH)₂ solution with flow rate of 3-5 ml/min.

3. The method according to claim 1-2, **wherein** in step f), the solution whose pH value is adjusted with NH₄OH is mixed for 15-30 minutes.

4. The method according to claim 1-3, **wherein** in steps g) and i), the solution is preferably left for resting preferably for duration of 12-28 hours, particularly preferably for 15-24 hours.

5. The method according to claim 1-4, **wherein** in step i), preferably 2-4% [(NH₄)₂SO₄] solution is used.

6. The method according to claim 1-5, **wherein** in step k), the powder obtained in step k) is dried in stove at a temperature between 70 and 100°C.

## Patentansprüche

1. Verfahren zur Herstellung von Beta-Tri-Calciumphosphat, wobei das Verfahren **dadurch gekennzeichnet ist, dass** es folgende Schritte umfasst:
a. Bereitstellen einer Ca(OH)₂ Lösung durch Zugabe von destilliertem Wasser zu Ca(OH)₂,
b. Bringen der Reaktionstemperatur auf 25-35°C und vorzugsweise auf 30°C und Fortsetzen des Mischens,
c. Einstellen des pH-Werts der Reaktion auf einen Wert zwischen 7-9 durch Verwendung von HCl-Säure,
d. Zugeben von destilliertem Wasser zu [(NH₄)₂HPO₄] und Bereitstellen einer vollständig auflösenden und vorbereitenden [(NH₄)₂HPO₄]-Lösung,
e. Zugeben [(NH₄)₂HPO₄]-Lösung zur Ca(OH)₂,
f. Bringen des pH-Werts der Reaktion auf einen Wert zwischen 7-9 mittels der NH₄OH-Lösung und Mischen und Beenden der Reaktion danach,
g. Lassen der erhaltenen Lösung zwischen 8 und 30 Stunden reifen,
h. Zugeben von destilliertem Wasser zu [(NH₄)₂SO₄] und Bereitstellen einer 3%igen [(NH₄)₂SO₄]-Lösung,
i. Dekantieren der gereiften Lösung und Zugeben von [(NH₄)₂SO₄]-Lösung und Lassen für 8-30 Stunden ohne Mischen reifen,
j. Filtrieren der erhaltenen Lösung und Waschen mit reichlich Wasser, so dass der pH-Wert der Lösung zwischen etwa 6 und 8, vorzugsweise 7, liegt,
k. Trocknen des gewonnenen feuchten Pulvers im Ofen,
l. Sintern des getrockneten Pulvers für 1 Stunde bei einer Temperatur zwischen 900 und 1500°C,
m. Erhalten des Endprodukts durch sofortiges Abkühlen des Produkts am Ende des Sinterprozesses.

2. Verfahren nach Anspruch 1, **wobei** in Schritt e) die [(NH₄)₂HPO₄]-Lösung der Ca(OH)₂-Lösung mit einer Durchflussrate von 3-5 ml/min zugesetzt wird.

3. Verfahren nach Anspruch 1-2, **wobei** in Schritt f) die Lösung, deren pH-Wert mit NH₄OH eingestellt wird, 15 bis 30 Minuten lang gemischt wird.

4. Verfahren nach Anspruch 1-3, **wobei** in den Schritten g) und i) die Lösung vorzugsweise für eine Dauer von 12-28 Stunden, insbesondere vorzugsweise für 15-24 Stunden, ruhen gelassen wird.

5. Verfahren nach Anspruch 1-4, **wobei** in Schritt i) vorzugsweise eine 2-4%ige [(NH4)25O4]-Lösung verwendet wird.

6. Verfahren nach Anspruch 1-5, **wobei** in Schritt k) das Pulver, das in Schritt k) erhalten wurde, in einem Ofen bei einer Temperatur zwischen 70 und 100°C getrocknet wird.

## Revendications

1. Méthode à utiliser dans la production de phosphate beta-tri-calcique, ladite méthode est **caractérisée en ce qu'**elle comprend les étapes suivantes :
a. Préparer la solution de Ca(OH)z en ajoutant de l'eau distillée à Ca(OH)₂,
b. Porter la température de réaction à 25-35°C et de préférence, à 30°C et continuer à mélanger,
c. Ajuster le pH de la réaction entre 7-9 en utilisant de l'acide HCl,
d. Ajouter de l'eau distillée à [(NH₄)₂HPO₄] et fournir une dissolution complète et préparer une solution de [(NH₄)₂HPO₄],
e. Ajouter la solution de [(NH₄)₂HPO₄] à la solution de Ca(OH)₂,
f. Porter le pH de la réaction entre 7-9 au moyen d'une solution de NH₄OH et mélanger et terminer la réaction ensuite,
g. Laisser vieillir la solution obtenue pendant 8 et 30 heures,
h. Ajouter l'eau distillée à [(NH₄)₂SO₄] et préparer une solution de [(NH₄)₂SO₄] à 3%,
i. Décanter la solution vieillie et ajouter une solution de [(NH₄)₂SO₄] et laisser vieillir pendant 8-30 heures sans mélanger,
j. Filtrer la solution obtenue et la laver abondamment à l'eau de manière à ce que le pH de la solution soit compris entre 6 et 8 environ, de préférence 7,
k. Sécher la poudre humide obtenue dans le four,
l. Fritter la poudre séchée pendant 1 heure à une température comprise entre 900 et 1500°C,
m. Obtenir le produit final par refroidissement instantané du produit à la fin du processus de frittage.

2. Méthode selon la revendication 1, **dans laquelle** à l'étape e), la solution de [(NH₄)₂HPO₄] est ajoutée à la solution de Ca(OH)₂ avec un débit de 3-5 ml/min.

3. Méthode selon la revendication 1-2, **dans laquelle** à l'étape f), la solution dont la valeur pH est ajustée avec NH₄OH est mélangée pendant 15-30 minutes.

4. Méthode selon la revendication 1-3, **dans laquelle** aux étapes g) et i), la solution est de préférence laissée au repos pendant une durée de 12-28 heures, de préférence pendant 15-24 heures.

5. Méthode selon les revendications 1-4, **dans laquelle** à l'étape i), de préférence une solution de [(NH₄)₂SO₄] à 2-4% est utilisée.

6. Méthode selon les revendications 1-5, **dans laquelle** à l'étape k), la poudre obtenue à l'étape k) est séchée dans un four à une température comprise entre 70 et 100°C.
